# EUROPEAN PATENT APPLICATION

(11) **EP 4 710 872 A1**
(43) Date of publication of application: **18.03.2026**
(21) Application number: 24802838.3
(22) Date of filing: 29.04.2024
(51) Int. Cl.: A61B 17/221, A61F 2/01, A61M 1/38

(54) **PROTECTIVE SYSTEM**

(30) Priority: 06.05.2023 CN 202310508676; 06.05.2023 CN 202310505282
(71) Applicant: Shenzhen Intervenet Medical Technology Co., Ltd., Shenzhen, Guangdong 518042 (CN)
(72) Inventor: ZHANG, Wayne Wei, Shenzhen, Guangdong 518042 (CN); CHEN, Zhong, Shenzhen, Guangdong 518042 (CN); ZHANG, Xiong, Shenzhen, Guangdong 518042 (CN)
(74) Representative: Michalski Hüttermann & Partner Patentanwälte mbB
(86) International application number: PCT/CN2024/090582
(87) International publication number: WO 2024/230555

(57) **Abstract**

Disclosed is a protective system, including at least one protective sheath, a filtering and bypass device, and at least one return tube. The protective sheath includes an outer sheath tube and a filtering member arranged at a distal end of the outer sheath tube; the outer sheath tube is provided with a first tube cavity; the filtering member has a proximal opening and a distal opening; the distal opening is communicated with the first tube cavity by means of the proximal opening; the filtering and bypass device has an inlet and an outlet; the inlet is communicated with the first tube cavity; a proximal end of the return tube is communicated with the outlet; and a distal end of the return tube is used for returning blood filtered by the filtering and bypass device to a human body. The protective system of the present invention can protect branch vessels in deployment processes of branch stents and can return filtered blood back to the human body so as to reduce blood loss of patients in surgical processes.

## Description

### Technical Field

The present invention relates to the field of medical devices, and particularly, to a protective system.

### Background Art

During cardiovascular intervention, a surgical instrument is often, through a delivery sheath, delivered to a target lesion for surgeries, such as balloon dilation and stent dilation for intravascular stenosis, stent reconstruction for aortic dissections and aortic aneurysms, and aortic valve replacement for valve diseases. All of the above surgeries may cause thrombi or calcified fragments in blood vessels to be detached during treatment. Without protection, the detached emboli are flushed towards a distal end along with blood flow, causing distal vascular thrombosis. Vascular thrombosis in different parts of the body may lead to local pain, tissue necrosis, hemiplegia, dementia, and even a death risk to a patient.

Particularly in endovascular aortic arch repair, a high degree of atherosclerosis of the aortic arch of the patient and excessive endovascular aortic arch operations will increase a risk of atherosclerotic plaque detachment. In the prior art, a protective system is usually used at the distal end to intercept and capture detached emboli, to provide temporary protection for cerebral blood vessels. However, in the endovascular aortic arch repair involving branch vessels (brachiocephalic trunk, left common carotid artery, and left subclavian artery), not only the branch vessels are required to be protected, but also branch stents are required to be placed in the branch vessels. A protective device having a treatment function has not appeared at present yet.

In addition, in the prior art, a protective device is usually used at the distal end to intercept and capture the detached emboli, to provide temporary protection for cerebral blood vessels. However, because a lesion vascular segment and even the proximal end or distal end of the lesion site have different degrees of deformation, the protective device cannot achieve complete apposition to a vascular wall, and there is still a possibility that thrombi flow toward the cerebral blood vessels.

### Summary of the Invention

Based on this, it is necessary to provide a protective system having a treatment function in an interventional operation.

The present invention provides a protective system, including:
at least one protective sheath, where the protective sheath includes an outer sheath tube and a filtering member arranged at a distal end of the outer sheath tube, the outer sheath tube has a first tube cavity, the filtering member has a proximal opening and a distal opening, and the distal opening is communicated with the first tube cavity through the proximal opening;
a filtering and bypass device, where the filtering and bypass device has an inlet and an outlet, and the inlet is communicated with the first tube cavity; and
at least one return tube, where a proximal end of the return tube is communicated with the outlet, and a distal end of the return tube is configured to return blood filtered by the filtering and bypass device to a human body.

In an embodiment, the protective system further includes an inflow converter, the inflow converter includes at least one inflow adapter and an inflow opening, each inflow adapter is communicated with one outer sheath tube, and the inflow opening is communicated with the inlet of the filtering and bypass device.

In an embodiment, the protective system further includes an outflow converter, the outflow converter includes an outflow opening and at least one outflow adapter, the outflow opening is communicated with the outlet of the filtering and bypass device, and each outflow adapter is communicated with one return tube.

In an embodiment, the return tube is connected with the outer sheath tube side by side; and the return tube has a return opening, and the return opening is positioned on a proximal side of the filtering member.

In an embodiment, the protective system further includes a sliding connector, and the return tube is connected with the outer sheath tube through the sliding connector, such that the return tube can move in an axial direction of the outer sheath tube under the action of an external force.

In an embodiment, the return opening is inclined relative to the axial direction of the outer sheath tube, and one side, close to the outer sheath tube, of the return opening is closer to the distal end of the outer sheath tube compared with one side, away from the outer sheath tube, of the return opening.

In an embodiment, the filtering member includes a filter screen body, a filter screen framework supporting the filter screen body, and a pushing member;
the pushing member can be arranged inside the first tube cavity or outside the first tube cavity in a mode of axially moving relative to the outer sheath tube; and a distal end of the pushing member is connected with the filter screen framework.

In an embodiment, the filtering and bypass device includes a filtering cavity and a filter screen, the filtering cavity is communicated with the inlet and the outlet, and the filter screen is arranged in the filtering cavity; and

A density of the filter screen is smaller than that of the filtering member.

In an embodiment, the filtering and bypass device further includes an air outlet; the filtering cavity is connected with the inlet, the outlet, and the air outlet; and
the inlet is formed on an upper edge of an inflow side of the filtering cavity, the outlet is formed on a lower edge of an outflow side of the filtering cavity, and the air outlet is formed on an upper edge of the outflow side of the filtering cavity.

In an embodiment, the protective system further includes a pump, and the pump is arranged between the filtering and bypass device and the return tube.

The protective sheath of the protective system of the present invention is deployed in a branch vessel during the interventional operation in the direction of blood flow, a delivery device for branch stents can pass through the first tube cavity of the protective sheath and pass through the filtering member of the protective sheath to reach aortic arch, and in the branch stent deployment process, the filtering member intercepts emboli in the branch vessel. Meanwhile, the protective sheath is externally connected with the filtering and bypass device, blood carrying emboli passes through the first tube cavity of the protective sheath to enter the filtering and bypass device, and finally the blood filtered by the filtering and bypass device is returned back to a human body, so as to reduce blood loss of patients in the surgical processes.

### Brief Description of the Drawings

FIG. 1 is a top view of a protective system in Embodiment 1 of the present invention;
FIG. 2 is a schematic diagram of an aortic dissection involving brachiocephalic trunk, left common carotid artery, and left subclavian artery;
FIG. 3 is a schematic diagram after a stent is implanted in aortic arch;
FIG. 4 is an operating schematic diagram of a protective system in Embodiment 1 of the present invention;
FIG. 5 is a top view of an inflow converter in Embodiment 1 of the present invention;
FIG. 6 is a top view of a protective system in other embodiments of the present invention;
FIG. 7 is a top view of an outflow converter in Embodiment 1 of the present invention;
FIG. 8 is a schematic diagram of a protective sheath and a return tube in Embodiment 1 of the present invention;
FIG. 9 is a cross-sectional view of the protective sheath and the return tube in Embodiment 1 of the present invention;
FIG. 10 is a schematic diagram of a filtering and bypass device in Embodiment 1 of the present invention;
FIG. 11 is a top view of a protective system in Embodiment 2 of the present invention;
FIG. 12 is a local three-dimensional diagram of a protective sheath in Embodiment 3 of the present invention;
FIG. 13 is a testing schematic diagram of the protective sheath in Embodiment 3 of the present invention;
FIG. 14 is a sectional view of the protective sheath placed in a vessel in Embodiment 3 of the present invention (a first filter screen and a second filter screen are subjected to transparency treatment);
FIG. 15 is a left view of a first filtering member and a second filtering member in Embodiment 3 of the present invention (the first filter screen and the second filter screen are subjected to transparency treatment);
FIG. 16 is a schematic diagram of a length ratio of the first filtering member to the second filtering member in Embodiment 3 of the present invention (the second filtering member is subjected to transparency treatment);
FIG. 17 is a local three-dimensional diagram of the protective sheath in Embodiment 3 of the present invention (the first filter screen and the second filter screen are subjected to transparency treatment);
FIG. 18 is a three-dimensional diagram of a first framework and a second framework in Embodiment 3 of the present invention;
FIG. 19 is a local three-dimensional diagram of a protective sheath in Embodiment 4 of the present invention (a first filter screen and a second filter screen are subjected to transparency treatment);
FIG. 20 is a three-dimensional diagram of a first filter screen in Embodiment 5 of the present invention;
FIG. 21 is a three-dimensional diagram of a first filtering member in Embodiment 5 of the present invention;
FIG. 22 is a three-dimensional diagram of the first filtering member and a second filtering member in Embodiment 5 of the present invention;
FIG. 23 is a schematic diagram of a first ring-shaped structure and a second ring-shaped structure in Embodiment 6 of the present invention; and
FIG. 24 is a schematic diagram of a connecting structure for a first ring-shaped structure and a first filter screen in Embodiment 7 of the present invention.

### Detailed Description of the Invention

In order to make the objectives, technical solutions, and advantages of the present invention clearer, the following is a further detailed explanation of the present invention in conjunction with the accompanying drawings and embodiments. It may be understood that the specific embodiments described here are only intended to explain the present invention and are not intended to limit the present invention.

Unless otherwise defined, all technical and scientific terms used herein have the same meaning as those commonly understood by a person skilled in the art to which the present disclosure belongs. Terms used in the specification of the present disclosure herein are merely intended to describe objectives of the specific embodiments, but are not intended to limit the present invention. The term "and/or" used herein includes any and all combinations of one or more related listed items.

In order to more clearly describe structures of the present invention, "distal end" and "proximal end" are used as localizers. The localizers are conventional terms in the field of interventional medical devices. A "distal end" is defined as an end that is far away from an operator in a surgical process, and a "proximal end" is defined as an end that is close to the operator in the surgical process.

### Embodiment 1

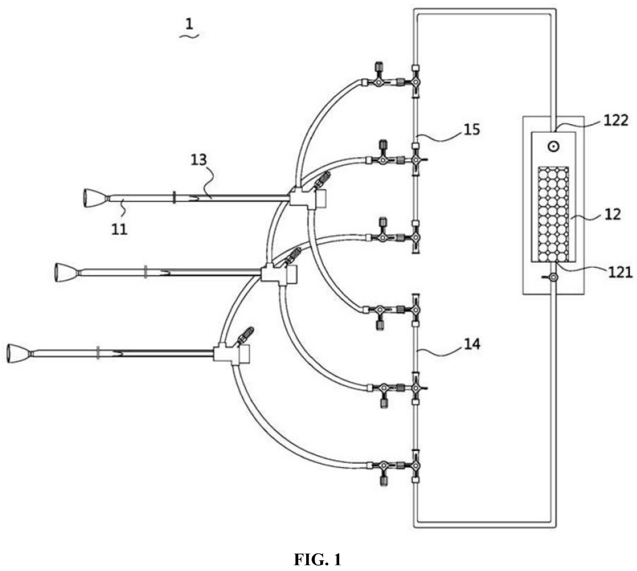

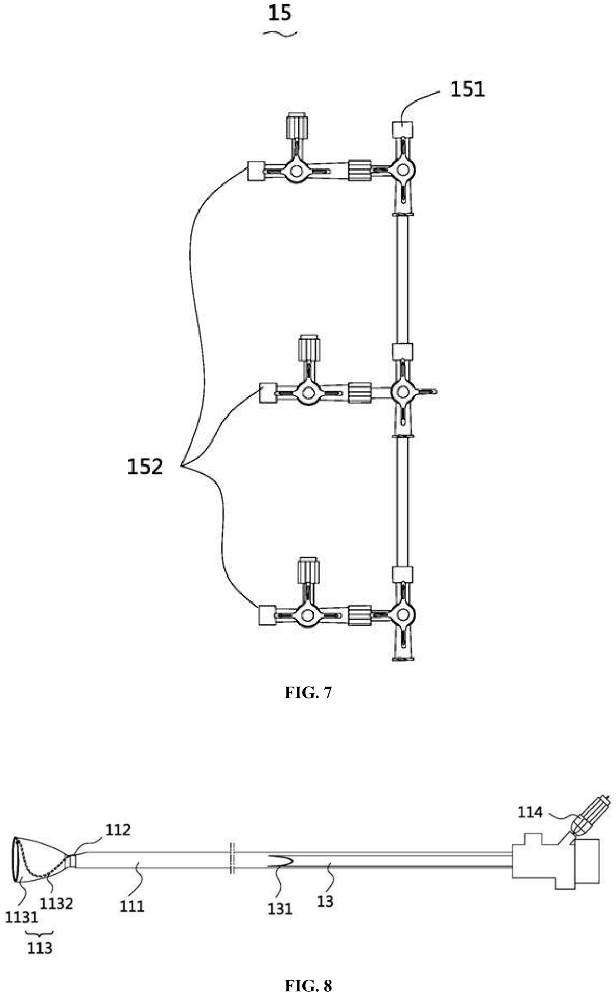

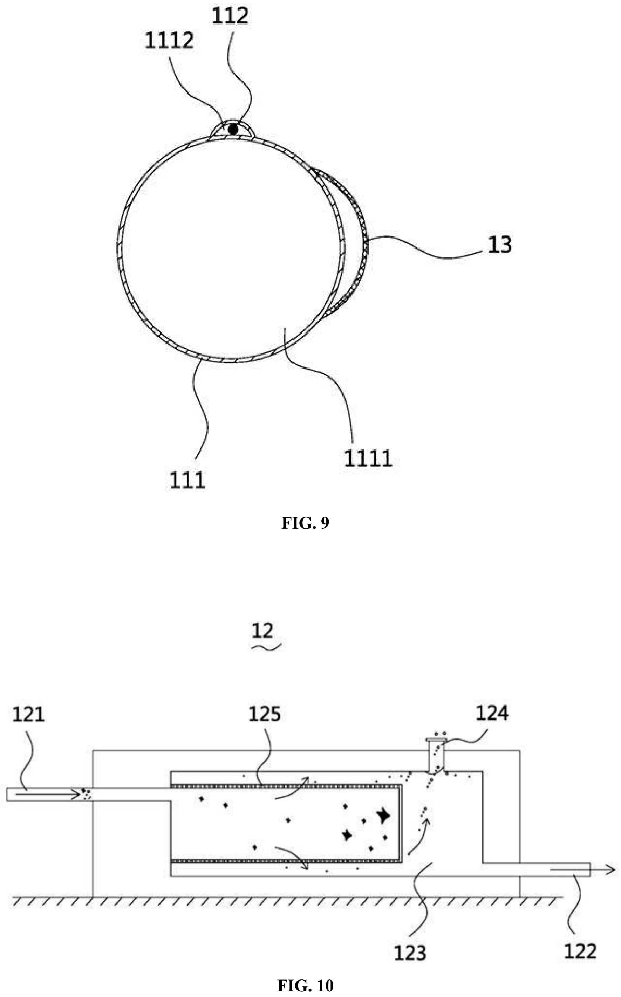

As shown in FIG. 1, a protective system 1 of this embodiment includes at least one protective sheath 11, a filtering and bypass device 12, and at least one return tube 13. As shown in FIG. 8 and FIG. 9, the protective sheath 11 includes an outer sheath tube 111 and a filtering member 113 arranged at a distal end of the outer sheath tube 111, the outer sheath tube 111 has a first tube cavity 1111, the filtering member 113 has a proximal opening and a distal opening, and the distal opening is communicated with the first tube cavity 1111 through the proximal opening.

Referring to FIG. 1 again, the filtering and bypass device 12 has an inlet 121 and an outlet 122, and the inlet 121 is communicated with the first tube cavity 1111.

A proximal end of the return tube 13 is communicated with the outlet 122, and a distal end of the return tube 13 is configured to return blood filtered by the filtering and bypass device to a human body.

The protective sheath 11 of the protective system 1 of the present invention is deployed in a branch vessel during the interventional operation in the direction of blood flow, a delivery device for a branch stent may pass through the first tube cavity 1111 of the protective sheath 11 and pass through the filtering member 113 of the protective sheath 11 to reach aortic arch, and in the branch stent deployment process, the filtering member 113 intercepts emboli in the branch vessel. Meanwhile, the protective sheath 11 is externally connected with the filtering and bypass device 12, blood carrying emboli passes through the first tube cavity 1111 of the protective sheath 11 to enter the filtering and bypass device 12, and finally blood filtered by the filtering and bypass device 12 is returned back to the human body, so as to reduce blood loss of patients in the surgical processes.

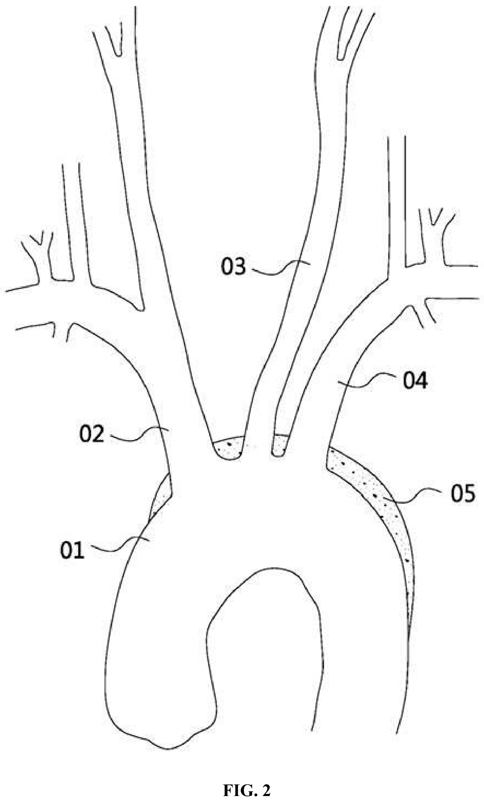

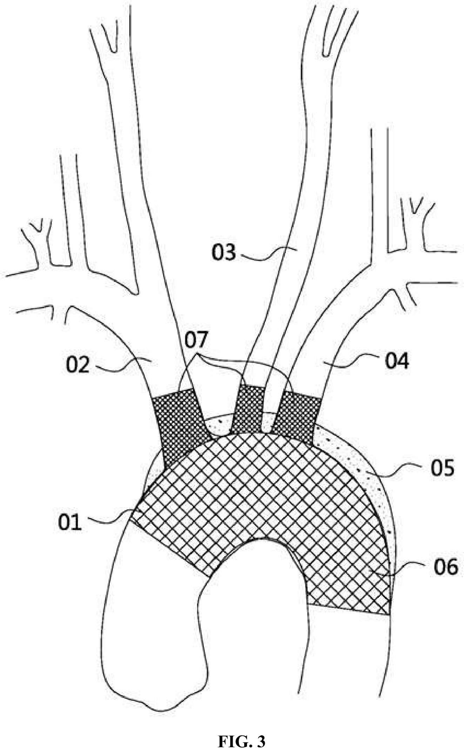

As shown in FIG. 2, when an aortic dissection 05 involves brachiocephalic trunk 02, left common carotid artery 03, and left subclavian artery 04, a conventional surgical method requires to deploy a main stent 06 in aortic arch 01 first, and the main stent 06 may be a pre-fenestrated stent, and also may be a common covered stent. When the main stent 06 is the pre-fenestrated stent, after the main stent 06 is deployed, branch stents 07 are delivered via a delivery device 2 through the brachiocephalic trunk 02, the left common carotid artery 03, and the left subclavian artery 04 respectively, and are deployed in the brachiocephalic trunk 02, the left common carotid artery 03, and the left subclavian artery 04 respectively. When the main stent 06 is the common covered stent, in-situ fenestration is required to be performed on the main stent 06 first, and then the branch stents 07 are delivered. As shown in FIG. 3, the main stent 06 is placed in the aortic arch 01, and the branch stents 07 are placed in the brachiocephalic trunk 02, the left common carotid artery 03, and the left subclavian artery 04, to repair the aortic arch 01 and ensure blood flow in the branch vessels. Whether the main stent 06 is the pre-fenestrated stent or the common covered stent, the branch stents 07 are required to be delivered and deployed through the branch vessels, and emboli generated in the process of delivering and deploying the branch stents 07 possibly flow toward a distal end along the brachiocephalic trunk 02, the left common carotid artery 03, and the left subclavian artery 04, causing irreversible damage to a patient.

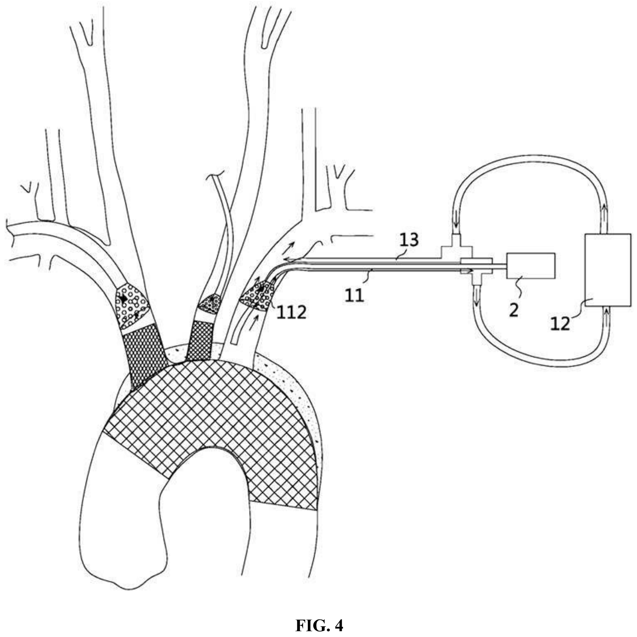

The protective system 1 of the present invention can achieve brain protection for the patient when the branch stents 07 are placed. Specifically, as shown in FIG. 4, the protective system 1 of this embodiment includes three protective sheaths 11; and before placement of the main stent 06, a protective sheath 11 is placed in each of the brachiocephalic trunk 02, the left common carotid artery 03, and the left subclavian artery 04, the filtering member 113 of the protective sheath 11 is allowed to be spread toward a proximal end, and the delivery device 2 for the branch stents 07 may pass through the outer sheath tube 111 and the filtering member 113 of the protective sheath 11 to deploy the branch stent 07 to a target position. In the process of delivering and deploying the main stent 06 and the branch stents 07, the filtering member 113 intercepts the emboli, and blood carrying emboli passes through the first tube cavity 1111 of the protective sheath 11 to enter the filtering and bypass device 12, and finally blood filtered by the filtering and bypass device 12 is returned back to the human body. It should be noted that in FIG. 4, a schematic diagram of connection between the protective sheaths 11 in the brachiocephalic trunk 02 and the left common carotid artery 03 and the filtering and bypass device 12 is not drawn.

In other embodiments, the protective system may include one or two protective sheaths. For example, in the aortic dissection 05 only involving the left subclavian artery 04, only one protective sheath is required to achieve protection of the left subclavian artery 04 and deployment of the branch stents 07. For example, in the aortic dissection 05 only involving the brachiocephalic trunk 02, only one protective sheath is adopted to achieve protection of the brachiocephalic trunk 02 and deployment of the branch stent 07, and a conventional filter screen structure only having a protective function is respectively placed in the common carotid artery 03 and the left subclavian artery 04 or at connection positions of the common carotid artery 03, the left subclavian artery 04, and the aortic arch 01, that is, the protective system of the present invention may be combined with protective devices with other structures for use. For example, in the aortic dissection 05 involving the common carotid artery 03 and the left subclavian artery 04, two protective sheaths are adopted to achieve protection of the common carotid artery 03 and the left subclavian artery 04 and deployment of the branch stents 07.

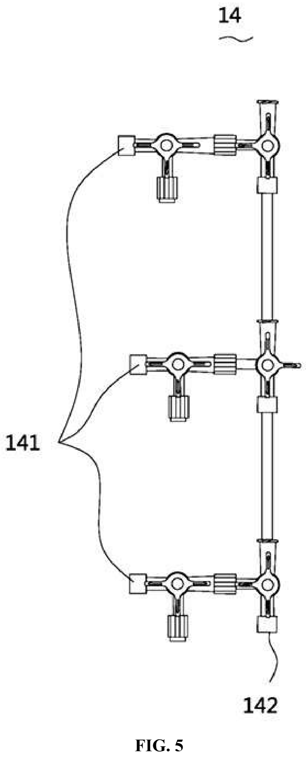

As shown in FIG. 1 and FIG. 5, the protective system 1 of this embodiment further includes an inflow converter 14, and the outer sheath tube is communicated with the filtering and bypass device 12 through the inflow converter 14; and the inflow converter is provided with at least one inflow adapter 141 and an inflow opening 142, each inflow adapter 141 is communicated with a proximal end of one outer sheath tube 111, and the inflow opening 142 is communicated with the inlet 121. In this embodiment, the inflow converter includes three inflow adapters 141, and the three inflow adapters 141 are connected to the three protective sheaths 11 in a one-to-one correspondence, such that blood from the three protective sheaths 11 is uniformly directed to the filtering and bypass device 12.

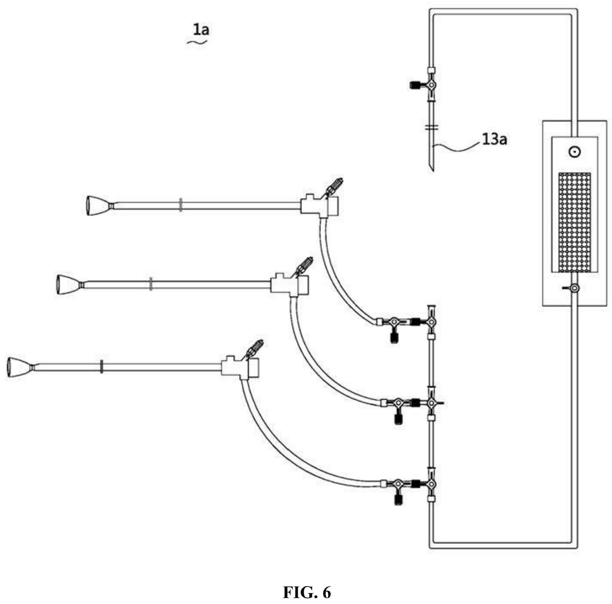

The return tube 13 may be connected to a vein of the patient, and also may be connected to an artery of the patient. As shown in FIG. 6, in other embodiments, when the return tube 13a is used for connecting a vein or one of branch vessels of the aortic arch 01 of the patient, the protective system 1a may only include one return tube 13a. In this embodiment, the protective system 1 includes three return tubes 13, and the three return tubes 13 are used for connecting the brachiocephalic trunk 02, the left common carotid artery 03, and the left subclavian artery 04 and return the blood filtered by the filtering and bypass device back to the brachiocephalic trunk 02, the left common carotid artery 03, and the left subclavian artery 04. Compared with a mode that filtered blood is returned back to the vein of the patient, a mode that the filtered blood is respectively returned back to the brachiocephalic trunk 02, the left common carotid artery 03, and the left subclavian artery 04 may improve perfusion of the brachiocephalic trunk 02, the left common carotid artery 03, and the left subclavian artery 04 during surgery and reduce a risk of ischemia of the patient during surgery.

When the delivery device for the branch stents passes through the first tube cavity 1111 and deploys the branch stents in the branch vessels, although a flow area of the first tube cavity 1111 becomes small, at this moment blood still may pass through the filtering member 113, to ensure blood supply for the branch vessels.

To achieve shunting of the three return tubes 13, as shown in FIG. 1 and FIG. 7, the protective system 1 of this embodiment further includes an outflow converter 15, the outflow converter 15 has an outflow opening 151 and three outflow adapters 152, the outflow opening 151 is communicated with the outlet 122, and each outflow adapter 152 is communicated with one return tube 13. In other embodiments, the outflow converter 15 may include one or two outflow adapters 152, and the number of the outflow adapters 152 is equal to the number of the return tubes 13.

When a patient with the aortic dissection 05 involving the brachiocephalic trunk 02, the left common carotid artery 03, and the left subclavian artery 04 undergoes an interventional operation, if the main stent 06 is the common covered stent, after deployment of the main stent 06, in-situ fenestration is performed at positions, corresponding to the three vessels, of the main stent 06. During the interventional operation, normal blood supply from the aortic arch 01 to brain may not be restored until fenestration is completed at least at positions corresponding to the brachiocephalic trunk 02 and the left common carotid artery 03; and however, blood supply for the left subclavian artery 04 may not be restored until fenestration is completed at a position corresponding to the left subclavian artery 04. As whole process is time-consuming, it is necessary to establish an extracorporeal circulation before delivery of the main stent 06.

However, the protective system 1 of this embodiment has the three return tubes 13, and the return tubes 13 are directly connected to the brachiocephalic trunk 02, the left common carotid artery 03, and the left subclavian artery 04, so during surgery, as long as one of three branch vessels, namely the brachiocephalic trunk 02, the left common carotid artery 03, and the left subclavian artery 04 is restored, blood is directed to the protective system 1, and then blood is simultaneously returned back to other two branch vessels, thereby shortening extracorporeal circulation time.

In this embodiment, as shown in FIG. 1 and FIG. 8, the return tube 13 is connected with the outer sheath tube 111 side by side, the return tube 13 has a return opening 131, and the return opening 131 is positioned on a proximal side of the filtering member 113. Therefore, as long as one puncture hole is made in each of the branch vessels, implantation of the branch stents 07 and return of the blood may be achieved, and injury to the patient is reduced. Refer to FIG. 4 again, due to presence of the filtering member 113, a blood pressure at the proximal end of the filtering member 113 is smaller than that at the distal end of the filtering member 113, so when the return opening 131 is positioned at the proximal end of the filtering member 113, which facilitates the smooth return of the filtered blood.

As shown in FIG. 8, the return opening 131 is inclined relative to an axial direction of the outer sheath tube 111, and one side, close to the outer sheath tube 111, of the return opening 131 is closer to a distal end of the outer sheath tube 111 compared with one side, away from the outer sheath tube 111, of the return opening 131. This configuration not only helps the return tube 13 and the outer sheath tube 111 to enter the puncture hole, but also may expand the flow area of the return opening 131, allowing blood in the return tube 13 to merge more quickly with blood in each of the branch vessels at the return opening 131.

In the interventional operation, a smaller outer diameter of a delivery sheath facilitates operation of the delivery sheath; and because the return tube 13 is connected with the outer sheath tube 111 side by side in this embodiment, an outer diameter of a portion, entering a vessel, of the protective system 1 is increased. To facilitate insertion of the outer sheath tube 111 into the vessel, a length of the return tube 13 inserted into the vessel is minimized, as long as the return opening 131 of the return tube 13 is completely positioned in the vessel. Therefore, for integrally formed or fixedly connected outer sheath tube and return tube, a distance is reserved between the return opening and the filtering member. The length of the distance depends on a length of a vessel in which the branch stent 07 is to be implanted and a position of the puncture hole, and in general, when a distance from the puncture hole to the aortic arch 01 is longer, the distance between the return opening and the filtering member is longer. To adapt to different branch vessels, the protective system 1 of this embodiment further includes a sliding connector 132, and the return tube 13 is connected with the outer sheath tube through the sliding connector 132, such that the return tube 13 may move in an axial direction of the outer sheath tube 111 under the action of an external force. During surgery, the outer sheath tube 111 may be placed first, then the return tube 13 is pushed into the vessel along the outer sheath tube 111, and the return opening 131 of the return tube 13 is completely positioned in the vessel. Specifically, as shown in FIG. 8, the sliding connector 132 is a ring-shaped body, and the ring-shaped body is sleeved outside the outer sheath tube and the return tube 13. The ring-shaped body is fixedly connected with the return tube 13, and the ring-shaped body and the outer sheath tube may slide relatively, so the return tube 13 may slide along the outer sheath tube 111. A distance from the ring-shaped body to the most proximal end of the return opening 131 falls within a range of 2 cm to 4 cm, and when the return tube 13 is pushed into the puncture hole, the ring-shaped body is abutted against a dilation sheath positioned in the puncture hole, to prevent the return tube 13 from further entering the vessel. Further, a locking structure is arranged on the ring-shaped body; after the return tube 13 is pushed in place, the locking structure locks the ring-shaped body to avoid movement of the return tube 13 during surgery; and the locking structure may be a releasable tie structure.

As shown in FIG. 9, a shape of the return tube 13 is fitted with a shape of an outer wall of the outer sheath tube 111, such that the return tube 13 may achieve apposition to the outer wall of the outer sheath tube 111.

Referring to FIG. 8 again, the filtering member 113 includes a filter screen body 1131 and a filter screen framework 1132 supporting the filter screen body 1131. The protective sheath 11 further includes a pushing member 112; the pushing member 112 may be arranged inside the first tube cavity 1111 or outside the first tube cavity 1111 in a mode of axially moving relative to the outer sheath tube 111; and a distal end of the pushing member 112 is connected with the filter screen framework 1132. In this embodiment, as shown in FIG. 9, the outer sheath tube 111 is further provided with a second tube cavity 1112, and the pushing member 112 is a guide wire and inserted into the second tube cavity 1112, to avoid interference to the delivery device 2 passing through the first tube cavity 1111. The filtering member 113 further includes a handle 114, and the handle 114 is connected to a proximal end of the pushing member 112 and used for retracting the filtering member 113. In other embodiments, the pushing member may also be integrally arranged in the first tube cavity. In some embodiments, the pushing member may also be a tubular body, the pushing member is arranged in a tube cavity, and the tube cavity for the pushing member provides a delivery channel for the delivery device 2.

As shown in FIG. 10, the filtering and bypass device 12 includes a filtering cavity 123 and a filter screen 125, the filtering cavity 123 is communicated with the inlet 121 and the outlet 122, and the filter screen 125 is arranged in the filtering cavity 123; and a density of the filter screen 125 is smaller than that of the filter screen body 1131.

Therefore, after deployment of the filtering member 113, a pressure difference between blood at a distal end of the filter screen body 1131 and blood at a proximal end of the filter screen body is large. In contrast, a pressure loss of blood, which enters the outer sheath tube, flows through the filtering and bypass device 12, and returns to the distal end of the filter screen body 1131, is small, and the blood remaining at a high pressure may return to a branch vessel at the proximal end of the filter screen body 1131 more smoothly.

As shown in FIG. 10, the filtering cavity 123 is connected with the inlet 121, the outlet 122, and an air outlet 124; and the inlet 121 is formed at an upper edge of an inflow side of the filtering cavity 123, the outlet 122 is formed at a lower edge of an outflow side of the filtering cavity 123, and the air outlet 124 is formed at an upper edge of the outflow side of the filtering cavity 123. Specifically, during surgery, the filtering and bypass device 12 is horizontally placed, the inlet 121 is higher than the outlet 122, and under the action of gravity, air emboli in blood are floated to the air outlet above the outlet 122 and discharged, and blood with a larger density flows out of the outlet 122 at a lower position.

### Embodiment 2

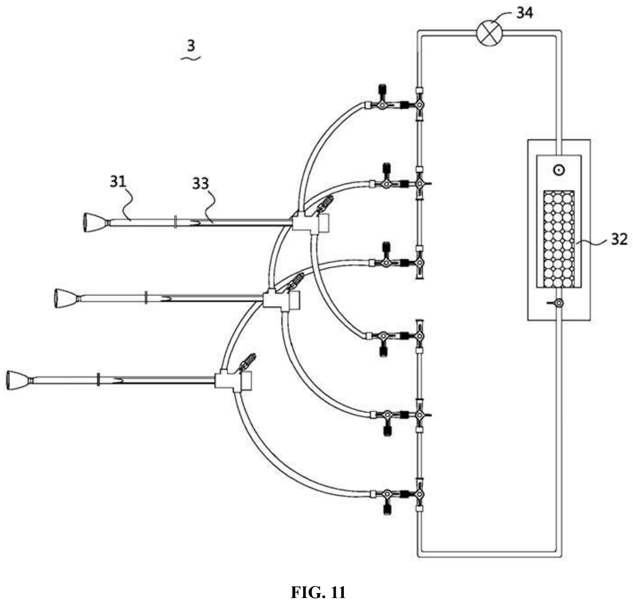

A main difference between Embodiment 2 and Embodiment 1 lies in that as shown in FIG. 11, a protective system 3 in Embodiment 2 further includes a pump 34, and the pump 34 is arranged between a filtering and bypass device 32 and a return tube 33. When a delivery device for branch stents enters a protective sheath 31, the delivery device occupies a flow area of a first tube cavity of the protective sheath 31, and to promote blood carrying emboli to smoothly flow through a gap between the delivery device and the protective sheath 31, and the pump 34 is arranged on a pipeline between the filtering and bypass device 32 and the return tube 33 to assist the blood in flowing toward a return opening of the return tube 33. The pump 34 may be an axial flow pump, a peristaltic pump, and a centrifugal pump, and preferably a peristaltic pump.

### Embodiment 3

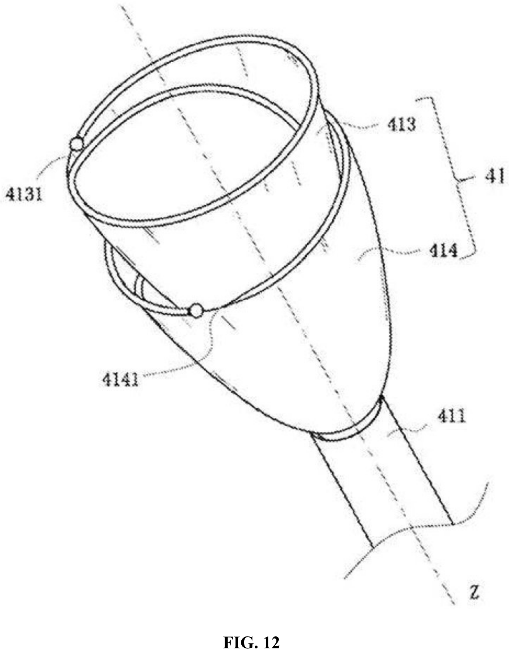

A main difference between Embodiment 3 and Embodiment 1 lies in that as shown in FIG. 12, a protective sheath 41 includes a first filtering member 413 and a second filtering member 414. Circumferential sizes of the first filtering member 413 and the second filtering member 414 are gradually reduced from a distal end to a proximal end; and the proximal end of the first filtering member 413 is arranged in the second filtering member 414, and the distal end of the first filtering member 413 exceeds the distal end of the second filtering member 414.

The first filtering member 413 is provided with a first deformation area 4131, and the second filtering member 414 is provided with a second deformation area 4141; and projections of the first deformation area 4131 and the second deformation area 4141 on a cross section of the protective sheath 41 are not overlapped, where the cross section is perpendicular to a central axis Z of the protective sheath 41. In this embodiment, the first filtering member 413 and the second filtering member 414 have the same shape and size, but orientations of the first filtering member 413 and the second filtering member 414 in a circumferential direction are not same, such that the projections of the first deformation area 4131 and the second deformation area 4141 on the cross section of the protective sheath 41 are not overlapped.

In the protective system of the present invention, the first filtering member 413 and the second filtering member 414 which are mutually independent and partially sleeved are arranged, and may provide double protection for a vessel. Meanwhile, the first filtering member 413 and the second filtering member 414 are provided with the first deformation area 4131 and the second deformation area 4141 in different orientations, such that the first filtering member 413 and the second filtering member 414 have different deformation degrees or different deformation directions when subjected to pressures in the same radial direction and in the same magnitude, and the protective sheath 41 can achieve better apposition to vessels of various shapes.

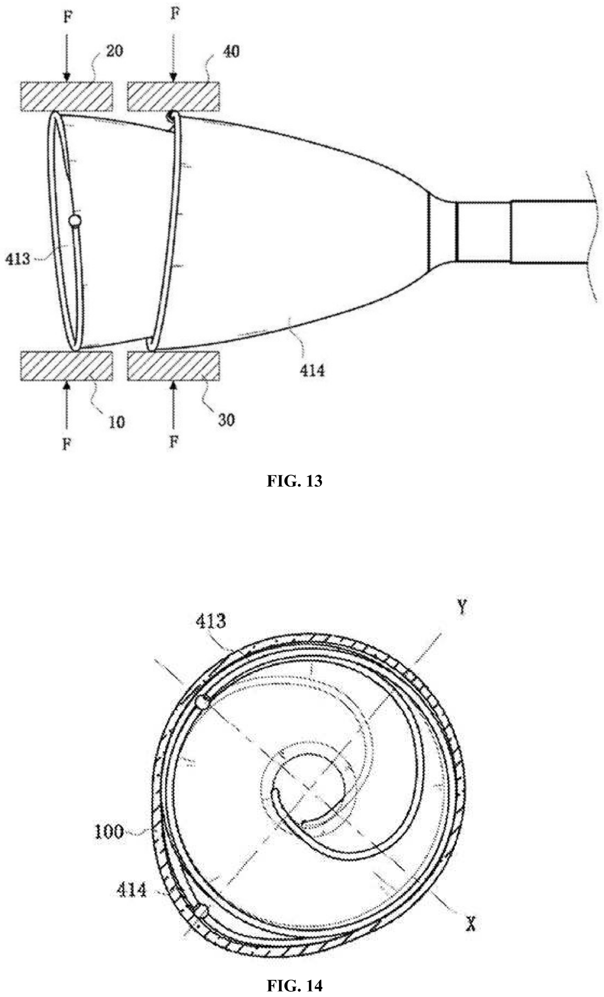

A flat test may be adopted to test diameter length changes of the first filtering member 413 and the second filtering member 414 under the action of pressures in the same radial direction and in the same magnitude, so as to test the deformation degrees of the first filtering member 413 and the second filtering member 414. For example, referring to FIG. 13, on a premise that the first filtering member 413 and the second filtering member 414 maintain in a free deployment state, parallel plates 10 and 20 are arranged on two opposite sides of the first filtering member 413, and radial acting forces F which are in the same magnitude but in opposite directions are respectively applied perpendicular to the plates 10 and 20; and the two parallel plates 10 and 20 maintain parallel to each other in a whole testing process, that is, the two parallel plates are always parallel to the central axis in the testing process. Similarly, parallel plates 30 and 40 are arranged on two opposite sides of the second filtering member 414, and radial acting forces F which are in the same magnitude but in opposite directions are respectively applied perpendicular to the plates 30 and 40.

If a radial length of the first filtering member 413 in a natural deployment state clamped between the plates is denoted as R1, a radial length variation of the first filtering member 413 under the action of the radial force F is defined as a difference in radial lengths before and after radial compression, and may be expressed as ΔR1, and a radial length variation ratio is expressed as ΔR1/R1. To ensure that the plates do not deform during the application of radical force, and ensure that the radial force may be uniformly applied to all places of the plates, a thickness of the plates is at least 5 mm. In a similar way, if a radial length of the second filtering member 414 in a natural deployment state clamped between the plates is denoted as R2, where R2 is equal to R1, a radial length variation of the second filtering member 414 under the action of the radial force F is defined as a difference in radial lengths before and after radial compression, and may be expressed as AR2, and a diameter length variation ratio is expressed as ΔR2/R2.

Based on the above testing conditions, under the action of the radial forces in the same radial direction and in the same magnitude, if AR1/R1 is greater than ΔR2/R2, or ΔR1 is greater than ΔR2, it indicates that under the radial acting force, the deformation degree of the first filtering member 413 is larger, otherwise, the deformation degree of the second filtering member 414 is larger.

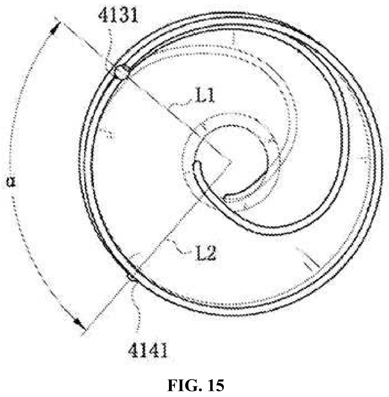

Specifically, the first filtering member 413 is required to have a certain support force to resist impact of blood flow. However, because of presence of deformation to some extent in a vascular segment of a lesion area or near a lesion area, for example in a vessel 100 in FIG. 14, a cross section of the vessel is not a regular circle, so the filtering member with a stronger support force has a problem of incomplete wall apposition. Therefore, the first deformation area 4131 is arranged, such that the first filtering member 413 may generate deformation to some extent to achieve better apposition to a vascular wall. Referring to FIG. 14, after deployment of the filtering member in the vessel 100, due to the presence of the first deformation area 4131, the first filtering member 413 extends and deforms in an X-axis direction more easily. In a similar way, due to the presence of the second deformation area 4141, the second filtering member 414 extends and deforms in a Y-axis direction more easily. Therefore, for example in FIG. 15, when the filtering members are subjected to a force in the X-axis direction from the vascular wall, deformation degrees of the first filtering member 413 and the second filtering member 414 are different, even if the first filtering member 413 does not achieve complete apposition to the vascular wall, the second filtering member 414, which extends and deforms in the Y-axis direction more easily, may achieve apposition to a protruding portion of the vascular wall and has a supplementary blockage function, that is, the filtering members may achieve self-adaptive apposition to the vascular wall under the pressure of the vascular wall.

It should be noted that in the first filtering member 413, due to the presence of the first deformation area 4131, the deformation degree and deformation direction of the first filtering member 413 vary depending on a relative position between a point where the pressure is applied to the first filtering member 413 and the first deformation area 4131. Similarly, due to the presence of the second deformation area 4141, the deformation degree and deformation direction of the second filtering member 414 vary depending on a relative position between a point where the pressure is applied to the second filtering member 414 and the second deformation area 4141. Therefore, a condition that the deformation degrees of the first filtering member 413 and the second filtering member 414 are the same at specific positions may occur. However, due to different orientations of the first deformation area 4131 and the second deformation area 4141 in the circumferential direction of the protective sheath, deformation shapes, namely, extension and protrusion directions, of the first filtering member 413 and the second filtering member 414 are not exactly the same, that is, the projections of the first filtering member 413 and the second filtering member 414 on the cross section of the protective sheath are not completely overlapped, and the filtering members may still adapt to a shape of the vessel, thereby improving the whole apposition of the filtering members.

As shown in FIG. 15, a perpendicular segment from a starting point of the first deformation area 4131 to the central axis of the protective sheath is a first segment L1; a perpendicular segment from a starting point of the second deformation area 4141 to the central axis of the protective sheath is a second segment L2; and an included angle α formed by projections of the first segment L1 and the second segment L2 on the cross section is 80°-100°. When the included angle α is smaller than 80°, the first deformation area 4131 and the second deformation area 4141 are too close, and when the first filtering member 413 and the second filtering member 414 are subjected to the radial forces in the same radial direction and in the same magnitude, extension and protrusion directions of the first filtering member 413 and the second filtering member 414 are also too close, that is, the projections of the first filtering member 413 and the second filtering member 414 on the cross section of the protective sheath 41 tend to be overlapped, and the first filtering member 413 and the second filtering member 414 may not have the supplementary blockage function. Similarly, if the included angle α is greater than 100°, when the first filtering member 413 and the second filtering member 414 are subjected to the radial forces in the same radial direction and in the same magnitude, the extension and protrusion directions of the first filtering member 413 and the second filtering member 414 are also too close. Preferably, when the included angle α is 90°, an overlapping area of the projections of the first filtering member 413 and the second filtering member 414 on the cross section of the protective sheath 41 is minimized, which is more favorable for improving an apposition degree of the protective sheath 41 to the vascular wall.

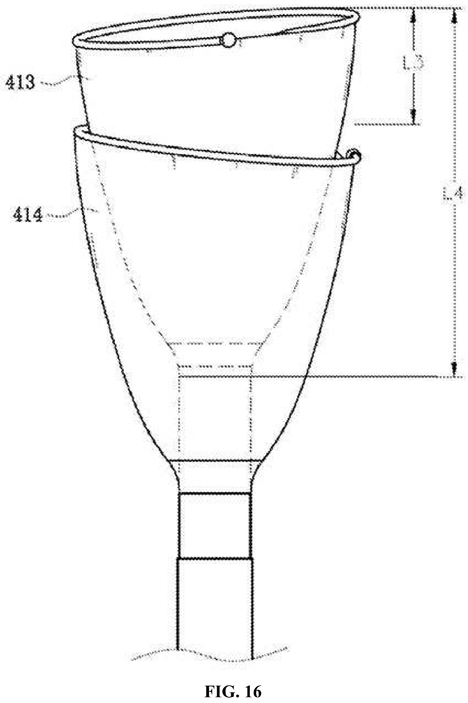

Because the first filtering member 413 and the second filtering member 414 are independently arranged parts, and only proximal ends of both the first filtering member 413 and the second filtering member 414 are connected to the pushing member 412, when the first filtering member 413 and the second filtering member 414 are pressed by the vascular wall, distal openings of the first filtering member 413 and the second filtering member 414 may be deformed independently, thereby achieving better apposition to the vascular wall. It should be noted that if a distance between the first filtering member 413 and the second filtering member 414 is too long, a vessel to be occupied is too long, which is unfavorable for implementation of surgery; and deformation shapes of vascular segments at long distances may be different, and the first filtering member 413 and the second filtering member 414 hardly have functions of blocking and intercepting thrombi for the second time. Therefore, in this embodiment, as shown in FIG. 16, a length L3 of a portion, exceeding the distal end of the second filtering member 414, of the first filtering member 413 is one fourth to one half of an overall length L4 of the first filtering member 413. Circumferential sizes of the first filtering member 413 and the second filtering member 414 are gradually reduced from the distal end to the proximal end, that is, an outer side of the proximal end of the first filtering member 413 provides a retraction space for the second filtering member 414, and even if the first filtering member 413 and the second filtering member 414 are partially sleeved, smooth retraction of the filtering member by the outer sheath tube 411 is not affected.

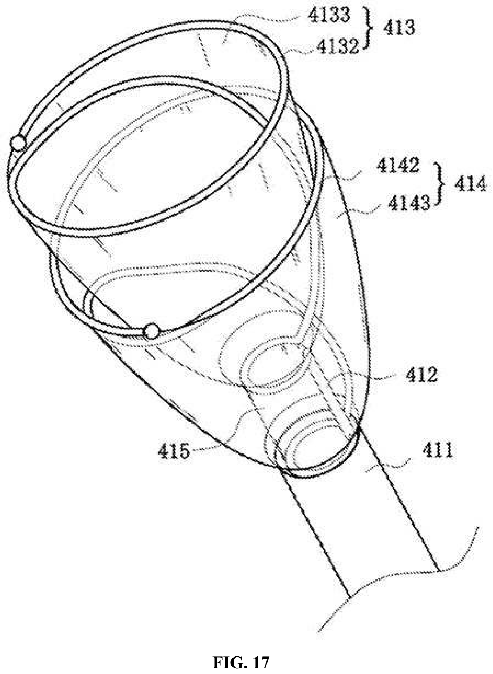

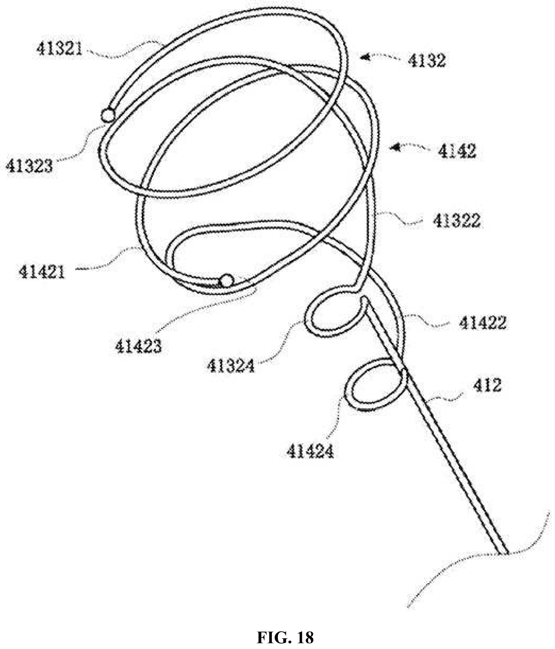

As shown in FIG. 17, the first filtering member 413 includes a first filter screen 4133 and a first framework 4132; and as shown in FIG. 18, the first framework 4132 includes a first ring-shaped structure 41321 and a first support structure 41322, the first ring-shaped structure 41321 is arranged along an edge of the first filter screen 4133, and the first support structure 41322 extends from the first ring-shaped structure 41321 to the proximal end of the first filtering member 413. In this embodiment, the second filtering 414 has a structure the same as the first filtering member 413, that is, the second filtering member 414 includes a second filter screen 4143 and a second framework 4142; the second framework 4142 further includes a second ring-shaped structure 41421 and a second support structure 41422; the second ring-shaped structure 41421 is arranged along an edge of a distal opening of the second filter screen 4143; and the second support structure 41422 extends from the second ring-shaped structure 41421 to the proximal end of the second filtering member 414. In other embodiments, the structure of the second filtering member 414 may be different from that of the first filtering member 413. The first framework 4132 and the second framework 4142 are made of materials with good biocompatibility and good elasticity, such as nickel-titanium alloy and stainless steel. A material for the first filter screen 4133 and the second filter screen 4143 is selected from a metal woven screen, a polymer material fiber woven or knitted screen, a polymer mixed metal woven or knitted screen, or a perforated polymer film, and a pore size of the filter screens is 0.1-0.5 mm.

In this embodiment, as shown in FIG. 18, the first ring-shaped structure 41321 has a first notch 41323, and the first notch 41323 forms the first deformation area 4131; and the second ring-shaped structure 41421 has a second notch 41423, and the second notch 41423 forms the second deformation area 4141. In other embodiments, the first deformation area and the second deformation area may also be a wavy segment, a segment with a small outer diameter, or an elastic segment made of elastic materials. A length of the first deformation area 4131 accounts for no more than 1/25 of a length of the first ring-shaped structure 41321, and a length of the second deformation area 4141 accounts for no more than 1/25 of the second ring-shaped structure 41421, because excessive long first deformation area 4131 and second deformation area 4141 may result in insufficient support strength of the first filtering member 413 and the second filtering member 414, which cannot meet an anchoring requirement.

Specifically, by taking the first filtering member 413 as an example, the first framework 4132 extends from a proximal end to a distal end of the first filter screen 4133, and finally is coiled on an edge of a distal opening of the first filter screen 4133 to form the first ring-shaped structure 41321 with the first notch 41323. In other embodiments, two ends of the notch 41323 of the first ring-shaped structure 41321 may be respectively connected with the first support structure 41322.

The first framework 4132 further includes a first proximal supporting ring 41324, and the first proximal supporting ring 41324 is arranged along an edge of a proximal opening of the first filter screen 4133; the second framework 4142 further includes a second proximal supporting ring 41424, and the second proximal supporting ring 41424 is arranged along an edge of a proximal opening of the second filter screen 4143; and the pushing member is a traction wire 412, and a distal end of the traction wire 412 is connected with the first proximal supporting ring 41324 and the second proximal supporting ring 41424 in sequence.

The pushing member in this embodiment is the traction wire 412, the distal end of the traction wire 412 is inserted into the outer sheath tube 51, a proximal end of the traction wire 412 penetrates the outer sheath tube 411 to reach an outer side of the outer sheath tube 411 through an opening in a wall of the outer sheath tube 411, thereby avoiding interference to a treatment device required to pass through the outer sheath tube 411, such as a stent delivery device. In other embodiments, the traction wire may be integrally inserted into the outer sheath tube; and alternatively, the outer sheath tube is a dual-tube cavity tube, one tube cavity is used for allowing the traction wire to penetrate, and the traction wire may be integrally inserted into the tube cavity, or partially inserted into the tube cavity.

An outer diameter of the traction wire 412 falls within a range of 0.3 mm to 0.35 mm, to ensure a strength required by supporting the first filtering member 413 and the second filtering member 414. A wire diameter of a material for manufacturing the first framework 4132 and the second framework 4142 falls within a range of 0.08 mm to 0.1 mm, so a space occupied by the first proximal supporting ring 41324 in the outer sheath tube 411 may be minimized, and the outer sheath tube 411 with the same inner diameter may accommodate a larger size delivery device.

As shown in FIG. 18, the first proximal supporting ring 41324 and the second proximal supporting ring 41424 are open rings, where an end portion of the first proximal supporting ring 41324 is connected with the first support structure 41322, and the other end portion of the first proximal supporting ring 41324 is connected with the traction wire 412; and one end portion of the second proximal supporting ring 41424 is connected with the second support structure 41422, and the other end portion of the second proximal supporting ring 41424 is connected with the traction wire 412. When the first filtering member 413 and the second filtering member 414 are required to be retracted, the traction wire 412 is pulled, and the first proximal supporting ring 41324 and the second proximal supporting ring 41424 may be deformed under the action of pulling of the traction wire 412, such that the first filtering member 413 and the second filtering member 414 are retracted into the outer sheath tube 411.

Further, referring to FIG. 17 again, a connecting column 415 is arranged between the first filtering member 413 and the second filtering member 414, the connecting column 415 is of a hollow columnar structure, a distal opening of the connecting column 415 is connected with the proximal opening of the first filter screen 4133, and a proximal opening of the connecting column 415 is connected with the proximal opening of the second filter screen 4143. The connecting column 415 enables stable connection between the first filtering member 413 and the second filtering member 414. A material for the connecting column 415 is selected from a metal woven screen, a polymer material fiber woven or knitted screen, a polymer mixed metal woven or knitted screen, or a perforated polymer film, and the connecting column 415 is connected with the first filtering member 413 and the second filtering member 414 by a welding or bonding method.

In some embodiments, one or more openings are formed in the connecting column, and emboli captured by the second filtering member may enter the first tube cavity through the openings, to avoid blockage of the second filtering member.

### Embodiment 4

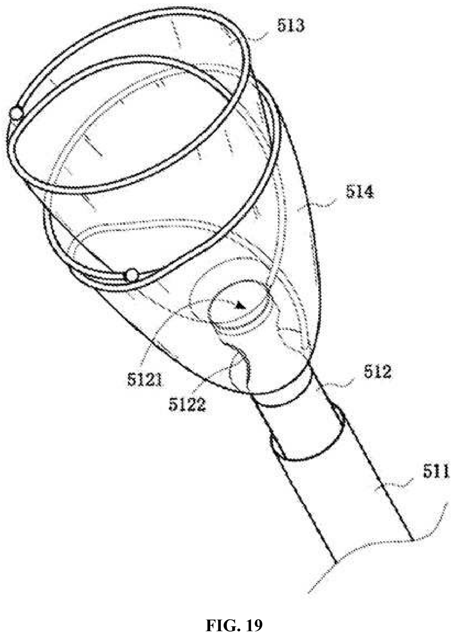

A main difference between Embodiment 4 and Embodiment 3 lies in that as shown in FIG. 19, a pushing member 512 of Embodiment 4 is of a hollow structure, and has a delivery tube cavity 5121. A first filtering member 513 and a second filtering member 514 are provided with a proximal opening respectively, the delivery tube cavity 5121 is communicated with a distal opening of the first filtering member 513 through the proximal opening of the first filtering member 513 to form a delivery channel, and the delivery channel is used for allowing a treatment device (such as a delivery device for branch stents) to pass through.

In this embodiment, a communicating hole 5122 is formed in the pushing member 512, and the communicating hole 5122 is formed between the first filtering member 513 and the second filtering member 514, and the delivery tube cavity 5121 is communicated with a distal opening of the second filtering member 514 through the communicating hole 5122. Through the communicating hole 5122, emboli captured by the second filtering member 514 may enter the delivery tube cavity 5121, to avoid blockage of the second filtering member 514.

Because the pushing member 512 has the delivery channel, during surgery, the protective system may be delivered to a distal end of a lesion site, the outer sheath tube is removed to release the first filtering member 513 and the second filtering member 514, and then a treatment device such as a stent delivery device, a filter delivery device, and a valve delivery device is delivered through the delivery channel. After completion of treatment, the treatment device is removed first, the pushing member 512 is pulled toward a proximal end to allow the first filtering member 513 and the second filtering member 514 to be retracted in an outer sheath tube 11, and finally the whole protective system is retracted. Specifically, the treatment device is placed in the delivery channel of the pushing member 512, and a plurality of control mechanisms for controlling movement of the treatment device are arranged on a handle (the control mechanisms are not shown in the figure). In other embodiments, the pushing member 512 may be a traction wire.

### Embodiment 5

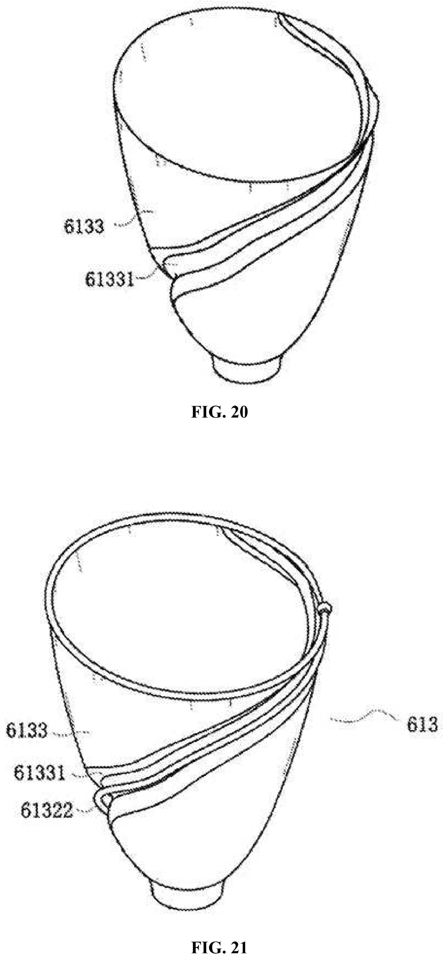

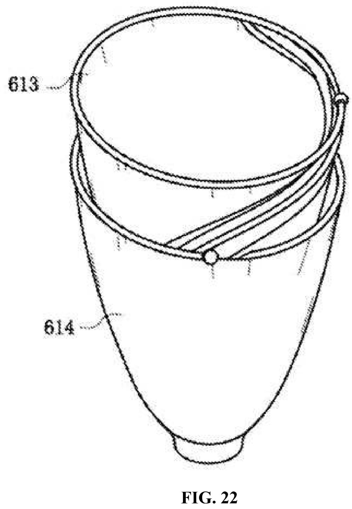

Referring to FIG. 20, a difference between this embodiment and Embodiment 4 lies in that a groove 61331 sunk toward an inner side of a first filter screen 6133 is formed on an outer surface of the first filter screen 6133 of the present invention. The groove 61331 helically extends from a distal end to a proximal end of the first filter screen 6133, and as shown in FIG. 21, a first support structure 61322 is arranged in the groove 61331. The filter screen 6133 is thin, so the groove 61331 appears as a bulge on the inner side of the first filter screen 6133, and the bulge has a guiding function in the first filter screen 6133, such that when blood flows through the first filter screen 6133, an eddy current effect is formed, to draw emboli to enter a bottom of a protective sheath and prevent a problem that the emboli escape or are stuck on a wall to prevent the blood from passing through micropores of the first filter screen 6133. However, plasma and healthy cells, which are smaller than the micropores of the first filter screen 6133, may pass through the micropores and flow toward a distal end. Further, a hollow pushing member connected with a first filtering member may be connected with a suction device or an extracorporeal circulation and filtering device, to timely suck thrombi out of the first filtering member. In this embodiment, a second filtering member has a structure the same as that of the second filtering member 514 in Embodiment 4, that is, in this embodiment, as shown in FIG. 22, the groove 61331 is only formed on a peripheral surface of the first filtering member 613. Therefore, in comparison with the protective sheath not provided with the groove 61331, a gap between the peripheral surface of the first filtering member 613 and an inner wall of the second filtering member 614 is larger. When a distal opening of the first filtering member 613 may not achieve apposition to a vascular wall, part of thrombi flow into the second filtering member 614 from the sufficiently large gap between the peripheral surface of the first filtering member 613 and an inner wall of the second filtering member 614, and then collected by the second filtering member 614. On the contrary, when the distal opening of the first filtering member 613 may not achieve apposition to the vascular wall, if the gap between the peripheral surface of the first filtering member 613 and an inner wall of the second filtering member 614 is too small, thrombi escaping from the first filtering member 613 are easily accumulated at a distal opening of the second filtering member 614 and hardly enter the second filtering member 614 smoothly, and during retraction of the protective sheath, and these thrombi possibly fall into a vessel again, thereby increasing a surgical risk.

In other embodiments, the groove is not formed near the distal opening of the first filtering member, that is, the groove extends from a proximal end to a distal end of the first filtering member, and a distal end of the groove exceeds the distal opening of the second filtering member but does not reach the distal opening of the first filtering member; and alternatively, a plurality of grooves are formed on the first filtering member. The above illustrated embodiments are not intended for limiting a shape and number of the groove, and grooves in other shapes and number may be formed on the first filtering member, as long as the thrombi may enter the second filtering member from the gap between the first filtering member and the second filtering member.

### Embodiment 6

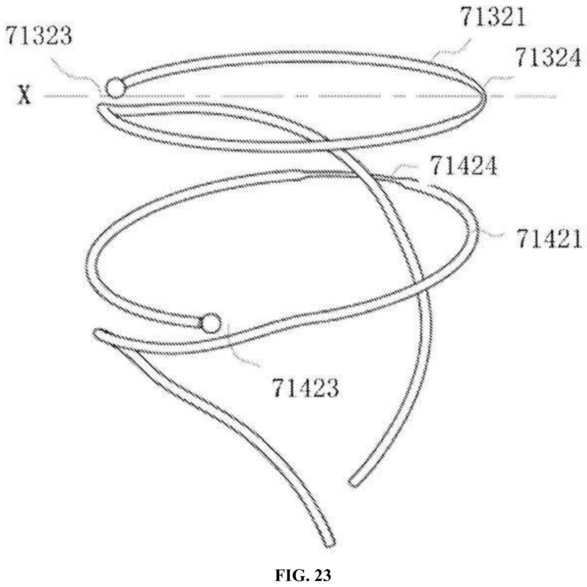

Referring to FIG. 23, a difference between this embodiment and Embodiment 1 lies in that a first deformation segment 71324 is arranged on a first ring-shaped structure 71321, and a projection of the first deformation segment 71324 and a projection of a first notch 71323 on a cross section of a protective sheath are opposite to each other in a circumferential direction; and a second deformation segment 71424 is arranged on a second ring-shaped structure 71421, and a projection of the second deformation segment 71424 and a projection of a second notch 71423 on the cross section of the protective sheath are opposite to each other in the circumferential direction. An opposite positional relationship in the circumferential direction is illustrated by taking the first ring-shaped structure 71321 as an example, and in comparison with a fact that the first notch 71323 and the first deformation segment 71324 are arranged at other positions of a distal opening of the first ring-shaped structure 71321, when the first notch 71323 and the first deformation segment 71324 directly faces each other in the circumferential direction, a circumferential distance between the first notch 71323 and the first deformation segment 71324 is the longest.

In this embodiment, an outer diameter of the first deformation segment 71324 is smaller than that of other portions of the first ring-shaped structure 71321, such that the first ring-shaped structure 71321 deforms more easily in the first deformation segment 71324. In other embodiments, the first deformation segment 71324 may be a wavy segment or an elastic connector.

By arranging the first deformation segment 71324 and in combination with the first notch 71323, the first ring-shaped structure 71321 extends and deforms in an X direction more easily. The second ring-shaped structure 71421 also extends and deforms in a specific direction more easily due to addition of the second deformation segment 71424, and a diagram is not given herein. For a seriously deformed lesion vessel, the filtering members in this embodiment may achieve better apposition to a vascular wall.

### Embodiment 7

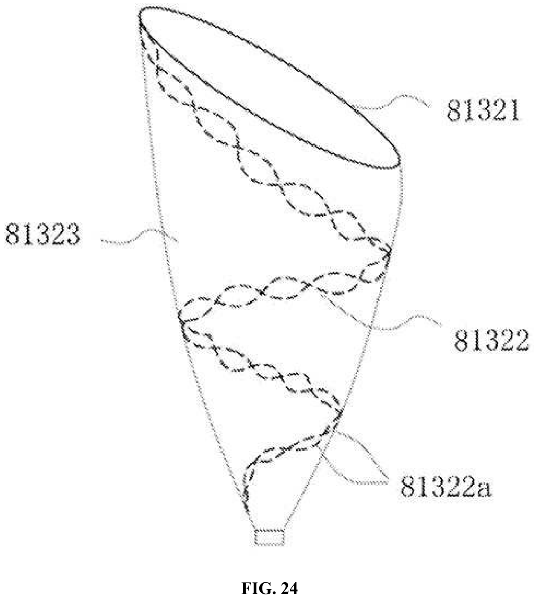

Referring to FIG. 24, a difference between this embodiment and Embodiment 1 lies in that a first ring-shaped structure 81321 is fixedly connected with a first filter screen 81333, a distal end of a first support structure 81322 is connected with the first ring-shaped structure 81321, and only a proximal end of the first support structure 81322 is fixedly connected with the first filter screen 81333; and a second ring-shaped structure is fixedly connected with a second filter screen, a distal end of a second support structure is connected with the second ring-shaped structure, and only a proximal end of the second support structure is fixedly connected with the second filter screen (the second filtering member of this embodiment is not shown in the figure).

The first ring-shaped structure 81321 is fixedly connected with the first filter screen 81333, to support a distal opening of the first filter screen 81333, and except the proximal end and the distal end, the rest portions of the first support structure 81322 may move relative to the first filter screen 81333. Similarly, except the proximal end and the distal end, the rest portions of the second support structure may move relative to the second filter screen, so when an outer sheath tube is utilized to retract a first filtering member and a second filtering member, the first support structure 81322 and the second support structure have a certain movable range, and may adapt to a narrow space inside the outer sheath tube. When the first filtering member and the second filtering member are released from the outer sheath tube, the first support structure 81322 and the second support structure restore a natural state to support the first filter screen 81333 and the second filter screen. As shown in FIG. 13, the first support structure 81322 has two branches 81322a, and the two branches 81322a are intertwined and reserve a certain interval, to expand a width of the first support structure 81322, and further to increase a contact area of the first support structure 81322 and the first filter screen and improve supporting capacity of the first support structure. Meanwhile, the first support structure 81322 is arranged on an inner wall of the first filter screen 81333, and the first support structure 81322 has the intertwined and spaced branches 81322a, so when thrombi in blood pass through the first support structure 81322, the thrombi undergo friction and collision with the first support structure 81322 for a plurality of times and may be decomposed and cut to some extent, and large thrombi are prevented from blocking the first filter screen 81333.

Technical features of the above embodiments may be combined freely, and to make descriptions concise, not all possible combinations of the technical features of the above embodiments are described. However, these combinations of the technical features should fall within the scope of the present invention as long as they are not contradictory.

The above embodiments only describe some implementation modes of the present invention specifically and in detail, but cannot be construed as a limitation to the patent scope of the present invention. It should be noted that those of ordinary skill in the art can further make several transformations and improvements without departing from the concept of the present invention. These transformations and improvements all fall within the protection scope of the present invention. Therefore, the protection scope of the present invention should be subject to the accompanying claims.

## Claims

1. A protective system, comprising:
at least one protective sheath, wherein the protective sheath comprises an outer sheath tube and a filtering member arranged at a distal end of the outer sheath tube, the outer sheath tube is provided with a first tube cavity, the filtering member has a proximal opening and a distal opening, and the distal opening is communicated with the first tube cavity via the proximal opening;
a filtering and bypass device, which has an inlet and an outlet, wherein the inlet is communicated with the first tube cavity; and
at least one return tube, wherein a proximal end of the return tube is communicated with the outlet, and a distal end of the return tube is used for returning blood filtered by the filtering and bypass device to a human body.

2. The protective system according to claim 1, wherein
the protective system further comprises an inflow converter, the inflow converter comprises at least one inflow adapter and an inflow opening, each inflow adapter is communicated with one outer sheath tube, and the inflow opening is communicated with the inlet of the filtering and bypass device.

3. The protective system according to claim 1, wherein
the protective system further comprises an outflow converter, the outflow converter comprises an outflow opening and at least one outflow adapter, the outflow opening is communicated with the outlet of the filtering and bypass device, and each outflow adapter is communicated with one return tube.

4. The protective system according to claim 1, wherein
the return tube is connected with the outer sheath tube side by side; and the return tube has a return opening, and the return opening is positioned on a proximal side of the filtering member.

5. The protective system according to claim 4, wherein
the protective system further comprises a sliding connector, and the return tube is connected with the outer sheath tube through the sliding connector, such that the return tube can move in an axial direction of the outer sheath tube under the action of an external force.

6. The protective system according to claim 4, wherein
the return opening is inclined relative to an axial direction of the outer sheath tube, and one side, close to the outer sheath tube, of the return opening is closer to the distal end of the outer sheath tube compared with one side, away from the outer sheath tube, of the return opening.

7. The protective system according to claim 1, wherein
the filtering member comprises a filter screen body and a filter screen framework supporting the filter screen body;
the protective sheath further comprises a pushing member; the pushing member may be arranged inside the first tube cavity or outside the first tube cavity in a mode of axially moving relative to the outer sheath tube; and a distal end of the pushing member is connected with the filter screen framework.

8. The protective system according to claim 1, wherein
the filtering and bypass device comprises a filtering cavity and a filter screen, the filtering cavity is communicated with the inlet and the outlet, and the filter screen is arranged in the filtering cavity; and
a density of the filter screen is smaller than that of the filtering member.

9. The protective system according to claim 1, wherein
the filtering and bypass device further comprises an air outlet;
a filtering cavity is connected with the inlet, the outlet, and the air outlet; and
the inlet is formed on an upper edge of an inflow side of the filtering cavity, the outlet is formed on a lower edge of an outflow side of the filtering cavity, and the air outlet is formed on an upper edge of the outflow side of the filtering cavity.

10. The protective system according to claim 1, wherein
the protective system further comprises a pump, and the pump is arranged between the filtering and bypass device and the return tube.

11. A protective system, comprising a protective sheath, wherein the protective sheath comprises an outer sheath tube, a pushing member capable of axially moving relative to the outer sheath tube, and a first filtering member and a second filtering member which are connected to a distal side of the pushing member;
circumferential sizes of the first filtering member and the second filtering member are gradually reduced from a distal end to a proximal end; the proximal end of the first filtering member is arranged in the second filtering member, and the distal end of the first filtering member exceeds the distal end of the second filtering member;
the first filtering member is provided with a first deformation area, and the second filtering member is provided with a second deformation area; and projections of the first deformation area and the second deformation area on a cross section of the protective sheath are not overlapped, wherein the cross section is perpendicular to a central axis of the protective sheath.

12. The protective system according to claim 11, wherein
a first segment is defined as a perpendicular segment from a starting point of the first deformation area to the central axis of the protective sheath;
a second segment is defined as a perpendicular segment from a starting point of the second deformation area to the central axis of the protective sheath; and
an included angle formed by projections of the first segment and the second segment on the cross section ranges from 80° to 100°.

13. The protective system according to claim 11, wherein
an axial length of a portion, exceeding the distal end of the second filtering member, of the first filtering member is one fourth to one half of a whole axial length of the first filtering member.

14. The protective system according to claim 11, wherein
the pushing member is of a hollow structure, and has a first tube cavity, the first filtering member is provided with a proximal opening and a distal opening which are opposite to each other, the first tube cavity is communicated with the distal opening of the first filtering member through the proximal opening of the first filtering member to form a delivery channel, and the delivery channel is used for allowing a treatment device to pass through.

15. The protective system according to claim 14, wherein
a communicating hole is formed in the pushing member, the communicating hole is formed between the first filtering member and the second filtering member, and the first tube cavity is communicated with an interior of the second filtering member through the communicating hole.

16. The protective system according to claim 11, wherein
the first filtering member comprises a first filter screen and a first framework, the first framework comprises a first ring-shaped structure and a first support structure, the first ring-shaped structure arranged along an edge of a distal opening of the first filter screen, and the first support structure extends from the first ring-shaped structure to the proximal end of the first filtering member.

17. The protective system according to claim 16, wherein
the first ring-shaped structure has a first notch, and the first notch is the first deformation area.

18. The protective system according to claim 17, wherein
a first deformation segment is arranged on the first ring-shaped structure, and a projection of the first deformation segment and a projection of the first notch on the cross section of the protective sheath are opposite to each other in a circumferential direction.

19. The protective system according to claim 16, wherein
the first ring-shaped structure is fixedly connected with the first filter screen, a distal end of the first support structure is connected with the first ring-shaped structure, and only a proximal end of the first support structure is fixedly connected with the first filter screen.

20. The protective system according to claim 16, wherein
in a natural state, a groove sunk toward an inner side of the first filter screen is formed on an outer surface of the first filter screen; the groove helically extends from a distal end of the first filter screen to a proximal end of the first filter screen; and the first support structure is arranged in the groove.
